# EUROPÄISCHE PATENTSCHRIFT

(11) **EP 1 673 111 B1**
(45) Veröffentlichungstag und Bekanntmachung des Hinweises auf die Patenterteilung: **23.06.2010**
(21) Anmeldenummer: 04790543.5
(22) Anmeldetag: 18.10.2004
(51) Int. Cl.: A61L 2/07, A61G 9/02, A61L 2/26

(54) **VERFAHREN ZUR KÜHLUNG VON REINIGUNGSGUT IN REINIGUNGS- UND DESINFEKTIONSAUTOMATEN**
METHOD FOR COOLING A CLEANED PRODUCT IN AUTOMATIC CLEANING AND DISINFECTING MACHINES
PROCEDE POUR REFROIDIR UN PRODUIT A NETTOYER DANS DES MACHINES DE NETTOYAGE ET DE DESINFECTION

(30) Priorität: 17.10.2003 DE 10348344
(43) Veröffentlichungstag der Anmeldung: 28.06.2006
(60) Teilanmeldung: 06024364.9
(73) Patentinhaber: MEIKO Maschinenbau GmbH & Co. KG, 77652 Offenburg (DE)
(72) Erfinder: GAUS, Bruno, 77654 Offenburg (DE); LEHMANN, Denis, 77799 Ortenberg (DE); NÄGER, Thomas, 77652 Offenburg (DE)
(74) Vertreter: Hörschler, Wolfram Johannes
(86) Internationale Anmeldenummer: PCT/EP2004/011709
(87) Internationale Veröffentlichungsnummer: WO 2005/037330

(56) Entgegenhaltungen:
- EP-A- 0 679 406
- GB-A- 1 340 399
- US-A- 5 271 893
- US-B1- 6 245 292

## Beschreibung

Die Erfindung bezieht sich auf ein Verfahren zur Kühlung von Reinigungsgut in Reinigungs- und Desinfektionsautomaten, die beispielsweise in Krankenhäusern sowie Pflegeheimen und dergleichen eingesetzt werden. Hier kommt es neben einer bequemen Handhabung und einer raschen Arbeitsweise auf gründliche Sauberkeit und Desinfektion, d.h. auf Hygiene besonders an.

### Stand der Technik

Aus DE 195 09 877 C2 ist eine Vorrichtung zum Reinigen und Desinfizieren von Steckbecken, Urinflaschen und dergleichen bekannt. Diese umfasst eine Kammer, eine Dampferzeugungskammer, einen Wasservorratstank und eine Wasserpumpe, wobei eine Wassereinlassleitung in die Kammer als Überlaufleitung des Wasservorratstanks ausgebildet ist. Die Dampferzeugungskammer und der Wasservorratstank sind nach dem Prinzip kommunizierender Gefäße verbunden, derart, dass selbst bei niedrigem Wasserniveau im Wasservorratstank ein Dampferzeuger in der Dampferzeugungskammer vollständig überflutet ist und eine Dampfeinlassleitung zwischen Dampferzeugungskammer und Kammer ebenfalls eine Überlaufleitung des Wasservorratstanks und der Wasservorratstank ein Überdruckventil für die Dampfeinlassleitung bildet.

Aus DE 198 31 950 C2 ist eine Maschine zum Reinigen und Desinfizieren von Pflegegeschirr bekannt. Das Pflegegeschirr wird über eine Tür in eine Kammer eingeführt, dort gehaltert und über Düsen mit Wasser oder Dampf besprüht. Es ist ein Wasserkasten, ein Wasserzulauf und ein Pumpensystem zur Einleitung vom Reinigungsflüssigkeit in eine Kammer vorgesehen. Ferner wird eine Heizeinrichtung für die Flüssigkeit und als Dampferzeuger eingesetzt, wobei der oberhalb des Flüssigkeitsspiegels des Dampfbereiches gebildete Dampf durch eine Dampfleitung in die Kammer geführt wird. Der Wasserkasten ist durch eine Trenneinrichtung bis unterhalb des niedrigsten Wasserspiegels unter Aufrechterhaltung einer Wasserverbindung in einen Hauptwasserkasten und einen Dampfbereich unterteilt. Die Heizeinrichtung ist als Dampferzeuger im Dampfbereich angeordnet.

Aus DE 198 38 180 C2 ist ein Verfahren und eine Vorrichtung zum Reinigen und Desinfizieren von Gefäßen bekannt. Mittels dieser Vorrichtung lassen sich insbesondere Steckbecken, Urinflaschen oder Absaugflaschen reinigen. Zunächst wird das betreffende Gefäß entleert und vorgespült durch kontinuierliches Besprühen mit frisch zugeführter Reinigungsflüssigkeit und kontinuierlichem Abführen verunreinigter Reinigungsflüssigkeit über einen Abfluss der Sprühvorrichtung. Das Besprühen mit Reinigungsflüssigkeit wird unterbrochen, wobei ein weiteres Abführen verunreinigter Reinigungsflüssigkeit erfolgt. Danach wird der Abfluss der Spülvorrichtung verschlossen. Es wird eine vorbestimmte Menge an Reinigungs- oder Desinfektionsflüssigkeit in die Spülkammer eingebracht. Danach erfolgt ein kontinuierliches Reinigen und Desinfizieren des Gefäßes durch Umwälzen und Besprühen mit der in der Spülkammer enthaltenden Reinigungs- oder Desinfektionsflüssigkeit. In die Spülkammer wird Heißdampf eingebracht, wobei beim Einbringen des Heißdampfes mittels einer Wasserstrahlpumpe oder einer elektrisch angetriebenen Pumpe feuchte Luft aus der Spülkammer abgesaugt wird.

Der Desinfektionsschritt erfolgt gemäß des aus DE 198 38 180 C2 bekannten Verfahrens mit Wasserdampf. Aufgrund des Einbringens von Wasserdampf in die Spülkammer heizt sich das Reinigungsgut bis zu einer eingestellten Temperatur auf. Am Ende des Desinfektionsschrittes ist die Spülkammer mit Dampf gefüllt und das Reinigungsgut auf eine Temperatur von beispielsweise 85°C aufgeheizt. Wird die Spülkammer nun durch den Bediener geöffnet, strömt der Dampf aus der Spülkammer in den Arbeitsraum. Je nach Desinfektionstemperatur, d.h. Temperatur des Wasserdampfes, besteht für den Bediener die Gefahr von Verbrühungen oder Verbrennungen durch den austretenden Dampf; ferner wird beim Öffnen der Tür der Spülkammer unnötig Feuchtigkeit in den Raum eingetragen, in dem der Reinigungs- und Desinfektionsautomat aufgestellt ist. Ferner ist es nicht ohne weiteres möglich, dass der Bediener das desinfizierte Reinigungsgut aus der Spülkammer sofort entnehmen kann, ohne sich der Gefahr einer Brandverletzung auszusetzen; es ist in der Regel eine gewisse Wartezeit erforderlich, bis das aufgeheizte Reinigungsgut durch die kühlere Raumluft abgekühlt ist.

Abhilfe wurde dadurch geschaffen, dass nach dem Desinfektionsschritt Wasser in die Spülkammer eingespritzt wird und das Waschgut auf diese Weise abgekühlt wird. Ferner wird durch das eingespritzte Wasser der Dampf in der Spülkammer kondensiert. Dieses zusätzlich eingetragene Wasser wird aus dem eingebauten Wasservorratstank des Reinigungs- und Desinfektionsautomaten entnommen. Nachteilig dabei ist, dass das Wasser aus dem Vorratstank mit Keimen belastet sein kann und damit die Gefahr einer Rückverkeimung von vorher desinfiziertem Spülgut besteht. Ein weiterer Nachteil durch die Kühlung durch nach dem Desinfektionsschritt eingetragenes Wasser liegt in einem erhöhten Wasserverbrauch. Ferner erschwert das Aufbringen des Wassers nach dem Desinfektionsschritt die selbsttätige Trocknung des aufgeheizten Reinigungsgutes.

Aus der EP 1 032 432 B1 ist ein Verfahren zur Dampfsterilisation zum Behandeln von Sterilgut bekannt, bei dem zunächst in einem Gasentfernungsschritt unter Einsatz von Dampf und einer Absaugvorrichtung Umgebungsluft aus einer Sterilisationskammer entfernt wird. Anschließend wird ein Dampfsterilisationsschritt durchgeführt, bei welchem Dampf in die Sterilisationskammer eingeleitet wird und dort bei einer vorgegebenen Temperatur und einem bestimmten Druck für eine bestimmte Zeit verbleibt. Anschließend wird ein nicht-kondensierbares Gas in die Kammer eingeleitet, wodurch der verbleibende Dampf aus der Kammer verdrängt wird. In einem nachfolgenden Schritt wird anschließend das nicht-kondensierbare Gas aus der Kammer abgepumpt und ein Unterdruck in der Kammer erzeugt, wobei das in der Kammer befindliche Gut getrocknet wird. Anschließend wird der Druck in der Kammer wieder auf den Umgebungsdruck angeglichen und das Gut aus der Kammer entfernt.

Das in der EP 1 032 432 B1 beschriebene Verfahren und die zur Durchführung des beschriebenen Verfahrens dargestellte Vorrichtung weisen jedoch gravierende Nachteile auf. So ist das Verfahren auf eine Vielzahl von zeitlich genau aufeinander abgestimmten Ventilöffnungs- und Abpumpvorgängen angewiesen, welche in der Praxis ein aufwändiges Pumpensystem und eine aufwändige elektronische Ventilsteuerung erforderlich machen. Das Verfahren und die Vorrichtung sind daher sehr zeitaufwändig und teuer, so dass sich das System in vielen Fällen, in denen eine kostengünstige und schnelle Desinfektion von beispielsweise Krankenhausbedarf wie z. B. Nachtgeschirr nicht sinnvoll einsetzen lässt. Es wäre vielmehr ein Verfahren und eine Vorrichtung wünschenswert, welche ohne den Einsatz aufwändiger Pumpensysteme oder elektronisch gesteuerter Absperrventile auskommen.

Weiterhin haben die in der EP 1 032 432 B1 beschriebene Vorrichtung und das beschriebene Verfahren den Nachteil, dass die Notwendigkeit eines Abwassersystems, welche insbesondere bei dem beschriebenen Krankenhausbedarf besteht, nicht ausreichend berücksichtigt ist. Da das beschriebene Verfahren auf aufwändige Vakuumschritte angewiesen ist, muss das Abwassersystem von der eigentlichen Sterilisationskammer durch ein Kesselbodenventil getrennt werden, da sonst Flüssigkeit in das Pumpensystem gelangen würde. Das System ist daher lediglich für geringere Abwassermengen geeignet, nicht beispielsweise für eine unmittelbare Reinigung von Nachgeschirr. Die in der EP 1 032 432 B1 beschriebene Vorrichtung und das beschriebene Verfahren eignen sich also vorwiegend für die Sterilisation von kleineren medizinischen Geräten, wie beispielsweise Operationsbesteck.

Für den täglichen Krankenhausbedarf wäre es hingegen wünschenswert, ein Verfahren und ein System zur Verfügung zu stellen, welches auch größere Flüssigkeitsmengen problemlos handhaben kann, also über ein leistungsstärkeres Abwassersystem verfügt. Dabei muss jedoch eine Geruchsbelästigung durch die größeren Abwassermengen, welche in dem in der EP 1 032 432 B1 beschriebenen Vorrichtung, bei der Luft aus der Sterilisationskammer unmittelbar in die Umgebung abgelassen wird, unvermeidlich ist, vermieden werden.

### Darstellung der Erfindung

Angesichts der Nachteile der aus dem Stand der Technik bekannten Lösungen liegt der Erfindung die Aufgabe zugrunde, die Gefahr der Rückverkeimung von desinfiziertem Reinigungsgut zu vermeiden, den Wasserverbrauch des Reinigungs- und Desinfektionsautomaten pro Arbeitszyklus zu senken sowie die Arbeitsbedingungen für den Bediener des Reinigungs- und Desinfektionsautomaten zu verbessern.

Erfindungsgemäß wird diese Aufgabe durch die Merkmale des Patentanspruchs 1 gelöst.

EP-A-679 406 offenbart ein Verfahren gemäβ dem Oberbegriff des Anspruchs 1.

Gemäß der vorgeschlagenen Lösung laufen innerhalb des Reinigungs- und Desinfektionsautomaten die Programmschritte Spülen mit Kaltwasser, Vorreinigen mit Warmwasser (oder Kaltwasser) und Reinigung mit Klarspülung sowie thermische Desinfektion mit Wasserdampf in bekannter Weise ab. Nach der Desinfektion des Reinigungsgutes durch in die Kammer eingebrachten Wasserdampf wird erfindungsgemäß zwangsweise Luft aus der Umgebung, so zum Beispiel aus dem Arbeitsraum, in welchem der Reinigungs- und Desinfektionsautomat aufgestellt ist, in die Kammer eingeblasen. Bezogen auf die Bedingungen in der Kammer ist die Luft, die aus dem Arbeitsraum abgezogen wird, kalt und trocken. Bei Eintrag dieser Luft in die mit Wasserdampf gefüllte Kammer des Reinigungs- und Desinfektionsautomaten kondensiert der Dampf in dieser und das in der Kammer enthaltene Reinigungsgut kühlt ab. Die von außerhalb zugeführte Luft, beispielsweise Raumluft, wird zusammen mit restlichem Wasserdampf durch einen Abluftstutzen, welcher mit dem Abwassersystem des Reinigungs- und Desinfektionsautomaten verbunden sein kann, in das Abwassersystem abgeführt.

Um die Luftströmung innerhalb der Kammer des Reinigungs- und Desinfektionsautomaten zu beeinflussen, lassen sich Absperrelemente wie Schieber, Klappen und Ventile in die Leitungen von und zur Kammer einsetzen. Unter Kammer wird nachfolgend die Kammer eines Reinigungs- und Desinfektinsautomaten verstanden, in welcher das zu reinigende Gut behandelt wird. Die Absperrelemente in den Leitungen von und zur Kammer können beispielsweise als Rückschlagventil ausgebildet sein, welches zwischen dem die Luft eintragenden Gebläse und der Kammer eingesetzt werden kann. Ferner können die Absperrelemente beispielsweise ausgebildet als Klappen, Schieber oder Ventile dazu eingesetzt werden, in Betriebspausen des Reinigungs- und Desinfektionsautomaten vom Abwassersystem ausgehende Geruchsbelästigungen zu vermeiden.

Wird der in die Kammer eingetragene Luftstrom nach dem Niederschlagen des Wasserdampfes in dieser weiter aufrechterhalten, lässt sich ein zusätzlicher Effekt dahingehend erzielen, dass der eingetragene Luftstrom Feuchtigkeit von der Oberfläche des Reinigungsgutes und der Kammer aufnimmt und dieses trotz geschlossener Kammertür trocknet.

Durch das erfindungsgemäß vorgeschlagene Verfahren lässt sich eine Rückverkeimung des Waschgutes vermeiden. Die in die Kammer eingeblasene Raumluft, d.h. mit Raumluftfeuchte und mit Raumlufttemperatur, ist in Bezug auf das im Wassertank vorhandene Wasser keimfrei, so dass die Gefahr einer Rückverkeimung durch im Wasser enthaltene Keime ausgeschlossen ist. Das gemäß der bisherigen Lösung zusätzlich in die Kammer eingespritzte Wasser und der damit einhergehende zusätzliche Wasserverbrauch entfällt, da zum Niederschlagen des Wasserdampfes in der Kammer ein anderes Medium, nämlich Umgebungsluft eingesetzt wird. Ferner wird durch die erfindungsgemäß vorgeschlagene Lösung weitestgehend ausgeschlossen, dass Wrasen (Dampfschwaden) in den Arbeitsbereich nach dem Öffnen der Kammertür eintreten und die Arbeitsbedingungen des den Reinigungs- und Desinfektionsautomaten betätigenden Bedieners beeinträchtigen.

Ferner ist durch die erfindungsgemäß vorgeschlagene Lösung in vorteilhafter Weise gewährleistet, dass eine Trocknung des Reinigungsgutes innerhalb der Kammer bei geschlossener Tür abläuft und somit keine zusätzliche Feuchtigkeit in den Arbeitsraum, in welchem der Reinigungs- und Desinfektionsautomat aufgestellt ist, eingetragen werden kann. Durch das zwangsweise Einblasen von Luft in die Kammer nach dem Ende des thermischen Desinfektionsschrittes wird einerseits das Niederschlagen des Wasserdampfes erreicht und andererseits eine Abkühlung des in der geschlossenen Kammer enthaltenen, gereinigten und desinfizierten Reinigungsgutes herbeigeführt. Dabei kann durch die erfindungsgemäß vorgeschlagene Lösung erreicht werden, dass das gereinigte und desinfizierte Reinigungsgut auf eine Temperatur abgekühlt wird, so dass es von der Bedienungsperson aus der Kammer entnommen werden kann, ohne das diese beim Anfassen des Reinigungsgutes Verbrennungen oder Verbrühungen erleidet.

### Zeichnung

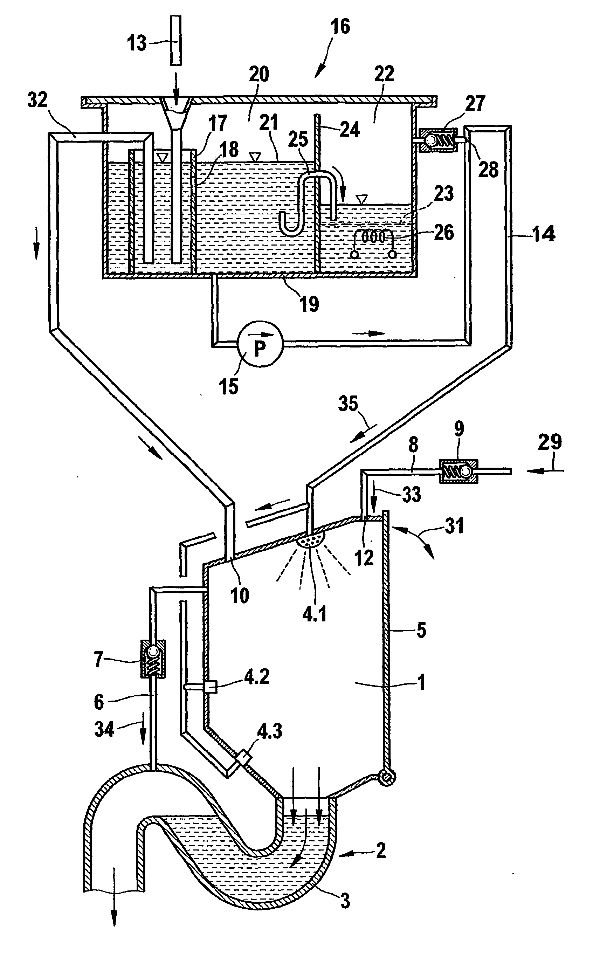

Anhand der Zeichnung wird die Erfindung nachstehend eingehender beschrieben:

Die einzige Figur zeigt in schematischer Weise den Aufbau sowie das Leitungsschema eines Reinigungs- und Desinfektionsautomaten, der mit dem erfindungsgemäß vorgeschlagenen Verfahren betreibbar ist.

In einem im Krankenhaus oder Pflegeheim eingesetzten Reinigungs- und Desinfektionsautomaten werden in einer Kammer 1 Steckbecken, Urinflaschen sowie andere Auffangbehältnisse für menschliche Ausscheidungen gereinigt und desinfiziert. Die Desinfektion dieser Behältnisse kann sowohl thermisch, d.h. mit Dampf, als auch chemisch erfolgen.

Dazu ist in den Reinigungs- und Desinfektionsautomaten in der Regel eine Kammer 1 ausgebildet, die von außen her über eine Tür 5 mit zu reinigenden Behältnissen beladen werden kann und durch welche die gereinigten desinfizierten Behältnisse aus der Kammer 1 auch wieder entnommen werden können.

Die Kammer 1 umfasst an ihrem unteren Ende einen einen Siphonbogen 3 enthaltenden Ablauf 2, über welchen die Reste menschlicher Ausscheidungen in ein Abwassersystem eingeleitet werden können. Der Ablauf 2 am unteren Ende der Kammer 1 mündet in ein Ablaufsystem. Zur Vermeidung von Geruchsbildung in der Kammer 1 dient der im Ablauf 2 ausgebildete Siphonbogen 3, der im Allgemeinen als Geruchssperre in Abwassersystemen vorgesehen ist, so auch an dem hier beschriebenen Reinigungs- und Desinfektionsautomaten. Die Kammer 1 des Reinigungs- und Desinfektionsautomaten ist durch eine schwenkbare Tür 5 zugänglich. Die Tür 5 ist um ein am unteren Ende der Tür angebrachtes Scharnier bewegbar und lässt sich in Öffnungs-/Schließrichtung 31 gemäß des in der Zeichnung eingetragenen Doppelpfeils bewegen. Die Kammer 1 ist über eine Wasser-/Dampf-Einheit 16 mit Dampf beaufschlagbar. In der Zeichnung sind Düsen 4.1, 4.2, 4.3 dargestellt, aus welchen Wasserdampf in die Kammer 1 eintreten kann. Diese sind in die Dachfläche der Kammer 1 integriert, können jedoch auch in deren seitlichen Begrenzungsflächen vorgesehen sein. Die Düsen 4.1, 4.2, 4.3, über welche Dampf in die Kammer 1 einleitbar ist, könnte ebenso gut an der rückwärtigen Begrenzungswand der Kammer 1 angebracht sein. In die Kammer 1 mündet ferner an einer Mündungsstelle 10 ein Sicherheitsüberlauf 32, über welchen Wasser aus der Wasser-/Dampf-Einheit 16 im oberen Bereich des Reinigungs- und Desinfektionsautomaten in die Kammer 1 überströmen kann und von dort in den Ablauf 2 gelangt. Der Sicherheitsüberlauf 32 könnte auch in den Ablauf 2 münden. Ferner befindet sich in der Dachfläche der Kammer 1 des Reinigungs- und Desinfektionsautomaten die Mündungsstelle 10 einer Zuluftleitung 8. Schließlich wird über eine Abluftleitung 6 Luft aus der Kammer 1 in den Ablauf 2 geführt, wobei die Mündungsstelle der Abluftleitung 6 hinter dem Siphonbogen 3 des Ablaufs 2 liegt.

Im oberen Bereich des Reinigungs- und Desinfektionsautomaten gemäß der Zeichnung ist eine Wasser-/Dampf-Einheit 16 aufgenommen. Der Wasser-/Dampf-Einheit 16 ist eine Wasserpumpe 15 zugeordnet, die unterhalb des Bodens 19 der Wasser-/Dampf-Einheit 16 angeordnet ist. Über die Wasserpumpe 15, die eine Druckerhöhung des Wassers herbeiführt, wird Wasser aus einem Wasserbehälter 20 der Wasser-/Dampf-Einheit 16 über einen Wasserzulauf 14 in die Kammer 1 gepumpt. Der Wasserbehälter 20 der Wasser-/Dampf-Einheit 16 wird über einen Kaltwasser- oder einen Warmwasserzulauf 13 versorgt. Je nach gebäudeseitigem Anschluss kann über den Wasserzulauf 13 entweder Kaltwasser in einem Temperaturbereich zwischen 10°C und 30°C in den Wasserbehälter 20 gefüllt werden oder, falls es sich um einen gebäudeseitigen Warmwasseranschluss handelt, Wasser mit einer Temperatur zwischen 45°C und 60°C in den Wasserbehälter 20 der Wasser-/Dampf-Einheit 16 einströmen. Auch eine Mischwasserbefüllung des Wasserbehälters 20 ist möglich. Das über den Wasserzulauf 13 zuströmende Warmwasser oder Kaltwasser strömt in einen Topf 17, der am Boden 19 der Wasser-/Dampf-Einheit 16 befestigt ist. Der Wasserspiegel im Topf 17 liegt im in der Zeichnung dargestellten Zustand unterhalb eines Überlaufes 18. Über den Überlauf 18 strömt das im Topf 17 über den Wasserzulauf 13 einströmende Wasser konstant in den Wasserbehälter 20 ein. Im Falle eines übermäßigen Wassereinlaufs strömt Wasser aus dem Topf 17 über den Sicherheitsüberlauf 32 durch die Mündungsstelle 10 in die Kammer 1 ein und strömt über den stets geöffneten Ablauf 2 in das Abwassersystem ab, so dass keine Wasserschäden in dem Raum auftreten können, in dem der Reinigungs- und Desinfektionsautomat aufgestellt ist.

Das aus dem Topf 17 über den Überlauf 18 in den Wasserbehälter 20 überströmende Wasser füllt den Wasserbehälter 20 bis zu einem Wasserpegel 21 an. Der Wasserbehälter 20 ist über eine Trennwand 24 von einem Dampferzeuger 22 der Wasser-/Dampf-Einheit 16 getrennt. Zur Befüllung des Dampferzeugers 20 mit Wasser erstreckt sich durch die Trennwand 24 eine Überströmleitung 25. Über die Überströmleitung 25 strömt Wasser in den Dampferzeuger 22 über. Nach dem Abpumpen stellt sich ein Pegel 23 im Dampferzeuger 22 ein. Zur Erwärmung des im Dampferzeuger 22 vorhandenen Wassers wird der im Dampferzeuger 22 enthaltene Wasservorrat über eine in der Zeichnung durch eine Wendel angedeutete Heizeinrichtung 26 erhitzt. Der bei der Erhitzung des Wassers entstehende Dampf wird über einen Leitungsabschnitt in die sich von der Wasserpumpe 15 aus erstreckende Zulaufleitung 14 zur Kammer 1 geführt. Der Leitungsabschnitt zwischen dem Dampferzeuger 22 und der Zulaufleitung 14 zur Kammer 1 ist durch ein Rückschlagventil 27 geschlossen. Aufgrund des Druckes des Wasserdampfes im Dampferzeuger 22 vermag das Rückschlagventil 27 zu öffnen, so dass an einer Mündungsstelle 28 Wasserdampf in die Zulaufleitung 14 zur Kammer 1 einströmen kann.

Über die Wasserpumpe 15 kann Wasser aus dem Wasserbehälter 20 auch über Sprühdüsen 4.2, 4.3, die im rückwandseitigen Bereich der Kammer 1 angeordnet sind, in diese eingespritzt werden. Die Sprühdüsen 4.1, 4.2, 4.3 können auch an den Seitenwänden oder an der der Kammer 1 zuweisenden Seite der die Kammer 1 verschließenden Türe angeordnet sein.

Der zur thermischen Desinfektion des in der Kammer 1 enthaltenen Reinigungsgutes benötigte Dampf wird bei entsprechender Dampferzeugung im Dampferzeuger 22 über das geöffnete Rückschlagventil 27 an der Mündungsstelle 28 in die Leitung 14 eingebracht und über diese entweder über in die Dachfläche der Kammer 1 integrierte Düsen 4.1, 4.2, 4.3 oder über an der Rückwand der Kammer 1 integrierte Düsen 4.1, 4.2, 4.3 zeitgleich in die Kammer 1 eingetragen.

Des Weiteren ist eine Belüftung der Kammer 1 über eine Zuluftleitung 8 möglich, die über ein Zuluftventil 9 freigebbar oder verschließbar ist. Über ein in der Zeichnung nicht dargestelltes Gebläse wird Zuluft 29, bei der es sich um relativ keimfreie Raumluft unter Raumluftbedingungen handelt, in die Kammer 1 eingetragen. Die Einströmrichtung der Raumluft 29 ist durch Bezugszeichen 33 kenntlich gemacht.

Das in der Kammer 1 des Reinigungs- und Desinfektionsautomaten ablaufende Programm kann gemäß der nachfolgend skizzierten Verfahrensschritte ablaufen, wobei es nicht zwingend ist, die nachfolgend aufgeführten Verfahrensschritte zu durchlaufen, sondern die nachfolgend wiedergegebenen Verfahrensschritte durchaus auch um andere Verfahrensschritte ergänzt werden können:

Zunächst wird die Kammer 1 mit Gefäßen, wie zum Beispiel Steckbecken, Urinflaschen, Nachttöpfen oder dergleichen bestückt, die mit menschlichem Blut oder anderen menschlichen Ausscheidungen verschmutzt sind, um diese zu reinigen und wieder zu verwenden. Nach Beschicken der Kammer 1 wird die Tür 5 verschlossen und es erfolgt ein erster Spülschritt mit Kaltwasser. Über die Sprühdüsen 4.2, 4.3 werden die im Inneren der Kammer 1 aufgenommenen Behältnisse mit kaltem Wasser in einem Temperaturbereich zwischen 10°C und 30°C ausgespült. An dem genannten Ausspülschritt mit Kaltwasser kann sich entsprechend des in der Kammer 1 ablaufenden Reinigungsprogrammes ein zweiter Reinigungsschritt anschließen, der mit Warmwasser durchgeführt werden kann, wobei dieses Wasser eine Temperatur zwischen 45°C und 60°C hat. Der zweite Vorreinigungsschritt kann hingegen auch mit Kaltwasser im oben genannten Temperaturbereich durchgeführt werden, um das Ankleben von Eiweiß-Rückständen, die in den menschlichen Ausscheidungen enthalten sein können, an zu reinigendem und zu desinfizierendem Reinigungsgut zu verhindern.

An den zweiten Vorreinigungsschritt der in der Kammer 1 enthaltenen Behältnisse kann sich eine Endreinigung mit Klarspülung anschließen. Dazu kann dem Waschwasser über eine in der Zeichnung nicht dargestellte feindosierende Pumpe ein Klarspülzusatz beigemischt werden. Der Klarspülzusatz wird in geringen Mengen dem aus dem Wasserbehälter 20 über die Wasserpumpe 15 beförderten Wasser, sei es Kaltwasser, sei es Warmwasser, beigemischt und auf die durch den ersten Spülschritt und den zweiten Vorreinigungsschritt gereinigten Behältnisse aufgebracht. An den Reinigungsschritt unter Zusatz eines Klarspülmittels schließt sich ein thermischer Desinfektionsschritt an. Beim thermischen Desinfektionsschritt wird die Entnahme von kaltem oder warmem Wasser aus dem Wasserbehälter 20 der Wasser-/Dampf-Einheit 16 unterbrochen und es wird im Dampferzeuger 22 erzeugter Wasserdampf über das Rückschlagventil 27 in die Zuleitung 14 zur Kammer 1 eingeleitet. Der Wasserdampf tritt entweder an in die Dachfläche 4 der Kammer 1 integrierten Düsen 4.1, 4.2, 4.3 aus oder an Düsen 4.1, 4.2, 4.3, welche in die rückwärtige Begrenzungswand der Kammer 1 integriert sind. Über den Wasserdampf erfolgt eine Desinfektion der durch die zuvor skizzierten Reinigungsschritte gereinigten Behältnisse innerhalb der Kammer 1. Während aller vorstehend aufgezählter Schritte befindet sich die Tür 5 der Kammer 1 stets im geschlossenen Zustand. Nach dem Desinfektionsschritt ist die Kammer 1 aufgrund der geschlossenen Tür 5 vollständig mit Dampf befüllt, der nur teilweise über die Abluftleitung 6 abzuströmen vermag. Nach dem Desinfektionsschritt wird in der Zuluftleitung 8 das Zuluftventil 9 geöffnet und Raumluft 29 unter Raumluftbedingungen in die Kammer 1 eingetragen. Im Vergleich zu dem in der Kammer 1 vorhandenen Wasserdampf ist die Raumluft 29 kalt und trocken. Aufgrund dessen kondensiert der in der Kammer 1 enthaltene Wasserdampf, und das in der Kammer 1 enthaltene Reinigungsgut kühlt ab. Die zugeführte Raumluft 29 wird zusammen mit dem restlichen, in der Kammer 1 enthaltenen Wasserdampf durch die Abluftleitung 6, in welcher das Abluftventil 7 geöffnet wird, in den Ablauf 2 geleitet und so aus der Kammer 1 abgeführt. Um die Zuluftströmung in die Kammer 1 zu beeinflussen, können zusätzliche Elemente wie Schieber, Klappen, Ventile 9 oder ähnliches in der Zuluftleitung 8 sowie in der Abluftleitung 6 aufgenommen sein. Wird das Zuströmen von Raumluft 29 in Zuströmrichtung 33 in die Kammer 1 aufrechterhalten, lässt sich ein weiterer zusätzlicher Effekt dahingehend erzielen, dass der zwangsweise in die Kammer 1 eingeblasene Zuluftstrom Feuchtigkeit von der Oberfläche des Reinigungsgutes und der Waschkammer aufnimmt und die Oberfläche des Reinigungsgutes sowie der Waschkammer trotz geschlossener Tür 5 trocknet. Dadurch kann erreicht werden, dass es nach Ablauf des Waschzyklus' bei einem Öffnen der die Kammer 1 verschließenden Tür 5 nicht zu einem Entweichen von Wasserdampfschwaden kommt, welche unter ungünstigen Umständen zu Verbrühungen beziehungsweise Verbrennungen an Händen und Armen des Bedieners führen können. Es hat sich herausgestellt, dass eine Betriebszeit des in der Zuluftleitung 8 aufgenommenen Gebläses für die Raumluft 29 bzw. Luft in Kammer 1 von etwa 40 s ausreichend ist, um den in der Kammer 1 nach der thermischen Desinfektion vorhandenen Wasserdampf niederzuschlagen. Eine Verlängerung der Gebläselaufzeit in der Zuluftleitung 8 kann dazu benutzt werden, zusätzlich eine Trocknung des in der Kammer 1 enthaltenen Reinigungsgutes herbeizuführen und dieses nicht nur auf eine Temperatur abzukühlen, die dessen Handhabung erlaubt, sondern auf darunter liegende Temperaturen so z.B. unterhalb von 65°C abzukühlen. Die Zeit des in der Raumluft 29 bzw. von einer anderen Luftquelle herstammende Luft in die Kammer 1 führenden Gebläses kann je nach den Erfordernissen eingestellt werden, wobei die Laufzeit jedoch mindestens solange bemessen sein sollte, dass sichergestellt ist, dass der in der Kammer 1 enthaltene Wasserdampf vollständig niedergeschlagen ist.

Wenn auch in der Zeichnung nicht näher dargestellt, erfolgt das Zumischen des Klarspülzusatzes über eine separate Pumpe, die zusätzlich zur Wasserpumpe 15 der Wasser-/Dampf-Einheit 16 zugeordnet sein kann, wobei die Leitung, die durch diese zusätzliche Dosierpumpe beaufschlagt wird, in die Leitung 14 zur Kammer 1 mündet.

Des weiteren ist es in Abwandlung des vorstehend skizzierten Reinigungsprogrammes durchaus möglich, fest anhaftende Rückstände wie zum Beispiel Salbenrückstände, die auf Sitzflächen der menschliche Ausscheidungen aufnehmenden Behälter verbleiben können, einen Salben entfernenden Zusatz der Reinigungsflüssigkeit zuzusetzen. Neben einem zusätzlichen Programmschritt, wie z.B. dem Zusatz eines salbenentfernenden Mittels zur Reinigungsflüssigkeit, können auch zusätzliche weitere Schritt in das Reinigungsprogramm eingebaut werden, wie es auch möglich ist, die vorstehend skizzierten Reinigungsschritte je nach Erfordernissen entweder mit Kaltwasser oder mit Warmwasser in den genannten Temperaturbereichen frei zu kombinieren.

Die erfindungsgemäß vorgeschlagene Maßnahme, nach Abschluss der thermischen Desinfektion mit Wasserdampf in die Kammer 1 Raumluft 29 unter Raumluftbedingungen einzutragen, gewährleistet, dass die Gefahr der Rückverkeimung des zuvor in der Kammer 1 gereinigten und desinfizierten Reinigungsgutes durch eventuell im Wasserbehälter 20 vorhandenes unsauberes Wasser deutlich reduziert ist. Andererseits bieten die erfindungsgemäß vorgeschlagenen Maßnahmen die Möglichkeit, das in der Kammer 1 enthaltene, gereinigte und desinfizierte Reinigungsgut abzukühlen, so dass es durch den Bediener ohne weiteres aus der Kammer 1 entnommen werden kann und bei entsprechender Verlängerung der Laufzeit des in der Zuluftleitung 8 enthaltenen Gebläses kann optional eine nahezu vollständige Trocknung des in der Kammer 1 enthaltenen Reinigungsgutes erreicht werden.

Da der Wasserbehälter 20 über dem gebäudeseitig gestellten Zulauf entweder mit Kaltwasser oder mit Warmwasser befüllt wird, ist eine Verkeimung dieses Wassers nicht völlig auszuschließen. Zur Durchführung des ersten Spülschrittes beziehungsweise des zweiten Vorreinigungsschrittes ist dieses Wasser jedoch geeignet, jedoch nicht zur Herbeiführung eines Rückkühlungseffektes des Reinigungsgutes, nachdem dieses im Wege der thermischen Desinfektion mit Wasserdampf desinfiziert worden ist. Ferner bietet die erfindungsgemäß vorgeschlagene Einleitung von Raumluft 29 nach Durchführung des Desinfektionsschrittes in die Kammer 1 den Vorteil, dass eine Einsparung von bisher gemäß des Standes der Technik bekannten Lösungen zur Rückkühlung eingesetzten Wassers möglich ist. Ferner wird durch die erfindungsgemäß vorgeschlagene Lösung aufgrund der geschlossen haltbaren Tür 5 der Kammer 1 ein Austreten von Wrasen in den Arbeitsbereich des Arbeitsraumes verhindert, in welchem der Reinigungs- und Desinfektionsautomat aufgestellt ist. Des Weiteren bietet die erfindungsgemäß vorgeschlagene Einleitung von Raumluft 29 nach der Durchführung des Desinfektionsschrittes mit Wasserdampf die Möglichkeit einer Trocknung des in der Kammer 1 enthaltenen Reinigungsgutes bei geschlossener Tür 5.

### Bezugszeichenliste

- 1: Kammer
- 2: Ablauf
- 3: Siphonboden
- 4.1: Düse
- 4.2: Düse
- 4.3: Düse
- 5: Tür
- 6: Abluftleitung
- 7: Abluftventil
- 8: Zuluftleitung
- 9: Zuluftventil
- 10: Mündung Sicherheitsüberlauf

- 12: Mündung Zulufteintrag
- 13: Kaltwasser/Warmwasser-Zulauf
- 14: Zuleitung zur Kammer 1
- 15: Wasserpumpe
- 16: Wasser-/Dampf-Einheit
- 17: Topf
- 18: Überlauf
- 19: Boden
- 20: Wasserbehälter
- 21: Wasserspiegel im Wasserbehälter 20
- 22: Dampferzeuger
- 23: Wasserspiegel im Dampferzeuger
- 24: Trennwand
- 25: Überströmleitung
- 26: Heizeinrichtung
- 27: Rückschlagventil
- 28: Mündungsstelle Dampfleitung
- 29: Raumluft

- 31: Öffnungs-/Schließrichtung
- 32: Sicherheitsüberlauf
- 33: Zuströmrichtung Zuluft
- 34: Abströmrichtung Abluft
- 35: Eintrittsrichtung Wasser/Dampf

## Patentansprüche

1. Verfahren zum Abkühlen von desinfiziertem Reinigungsgut, welches in einer Kammer (1) eines einen Ablauf (2) aufweisenden Reinigungs- und Desinfektionsautomaten aufgenommen ist, wobei die Desinfektion des Reinigungsgutes auf thermischem Wege erfolgt und innerhalb des Reinigungs- und Desinfektionsautomaten ein Wasch- oder Reinigungsprogramm mit variabler Programmschrittabfolge abläuft, mit nachfolgenden Verfahrensschritten:
a) die Endreinigung des in der Kammer (1) enthaltenen Reinigungsgutes erfolgt durch Wasser unter Zusatz von Hilfsstoffen,
b) das in der Kammer (1) enthaltene Reinigungsgut wird thermisch desinfiziert,
c) nach der thermischen Desinfektion des Reinigungsgutes wird Luft (29) zwangsweise in die geschlossene Kammer (1) eingebracht, und **dadurch gekennzeichnet, dass**
d) Abluft aus der geschlossenen Kammer (1) bei geschlossener Tür (5) der Kammer (1) durch eine Abluftleitung (6) mit einem Abluftventil (7) in den Ablauf (2) abgeleitet wird, wobei der Ablauf (2) einen Siphonbogen (3) aufweist, wobei die Ableitung der Abluft unter Umgehung eines als Sperre fungierenden Wasservolumens im Siphonbogen (3) erfolgt.

2. Verfahren gemäß Anspruch 1, **dadurch gekennzeichnet, dass** bei einer verlängerten Dauer eines Ableitvorganges von feuchter Abluft aus der Kammer (1) bei geschlossener Tür (5) eine zusätzliche Trocknung des in der Kammer (1) enthaltenen Reinigungsgutes erfolgt.

3. Verfahren gemäß Anspruch 1, **dadurch gekennzeichnet, dass** die nach dem Desinfektionsschritt in die Kammer (1) eingeleitete Luft Raumluft ist.

4. Verfahren gemäß Anspruch 1, **dadurch gekennzeichnet, dass** zur Strömungsführung von Zuluft beziehungsweise Abluft in der Abluftleitung (6) beziehungsweise in der Zuluftleitung (8) selbsttätige Absperrelemente (7, 9) vorgesehen sind.

5. Verfahren gemäß einem oder mehrerer der Patentansprüche 1 bis 4, **dadurch gekennzeichnet, dass** die durch die Zuluftleitung (8) der Kammer (1) zugeführte Luft durch einen Mikrofilter zur Verbesserung der Keimfreiheit geführt wird.

6. Vorrichtung zur Durchführung des Verfahrens gemäß einem oder mehrerer der Ansprüche 1 bis 5, **dadurch gekennzeichnet, dass** eine Kammer (1) von einer Wasser-/Dampf-Einheit (16) sowohl mit Kaltwasser oder Warmwasser als auch mit Wasserdampf beaufschlagbar ist, wobei der Kaltwasser/Warmwassereintrag über Sprühdüsen (4.1, 4.2, 4.3) erfolgt, die Kammer (1) über eine Abluftleitung (6) mit einem Ablauf (2) in Verbindung steht und die Kammer (1) eine Mündungsstelle (12) einer freigebbaren oder verschließbaren Zuluftleitung (8) aufweist, über welche die Kammer (1) im geschlossenen Zustand mit Luft beaufschlagbar ist.

7. Vorrichtung gemäß Anspruch 6 **dadurch gekennzeichnet, dass** die Mündungsstelle der Zuleitung (14), über welche die Kammer (1) zeitgleich über Düsen (4.1, 4.2, 4.3) mit Dampf beaufschlagbar ist, entweder an einer Dachfläche der Kammer (1) oder einer rückwärtigen Begrenzungswandung oder im unteren Bereich oder den seitlichen Begrenzungswänden der Kammer (1) ausgebildet ist.

8. Vorrichtung gemäß Anspruch 6, **dadurch gekennzeichnet, dass** in der mit der Kammer (1) verbundenen Leitungen (6, 8) für Zuluft oder Abluft selbsttätige Absperrelemente, die entweder federgesteuert oder gewichtgesteuert oder membrangesteuert oder differenzdruckgesteuert oder als Rückschlagventile (7, 9) ausgebildet sind, aufgenommen werden.

9. Vorrichtung gemäß Anspruch 8, **dadurch gekennzeichnet, dass** die Absperrelemente als zwangsgesteuerte Absperrelemente ausgebildet sind.

## Claims

1. Method for the cooling of cleaned and disinfected items contained in a chamber (1) of an automatic cleaning and disinfecting machine that has an outflow (2), the cleaned items being disinfected by heat, and a washing or cleaning program with a variable sequence of program steps being executed inside the automatic cleaning and disinfecting machine, said method comprising the following method steps:
a) the final cleaning of the items contained in the chamber (1) is carried out using water with addition of auxiliary agents,
b) the cleaned items contained in the chamber (1) are disinfected by heat,
c) after the heat disinfection of the cleaned items, air (29) is forcibly introduced into the closed chamber (1), and
**characterized in that**
d) with the door (5) of the chamber (1) closed, exhaust air is conveyed out of the closed chamber (1) into the outflow (2) via an exhaust air duct (6) having an exhaust air valve (7), the outflow (2) having a siphon bend (3), and the removal of the exhaust air bypassing a volume of water located in the siphon bend (3) and functioning as a barrier.

2. Method according to Claim 1, **characterized in that**, in the event of a prolonged duration of the removal of moist exhaust air out of the chamber (1) with the door (5) closed, an additional drying of the cleaned items contained in the chamber (1) takes place.

3. Method according to Claim 1, **characterized in that** the air introduced into the chamber (1) after the disinfection step is ambient air.

4. Method according to Claim 1, **characterized in that** automatic shut-off elements (7, 9) are provided in the exhaust air duct (6) and in the intake air duct (8), respectively, for controlling the flow of intake air and exhaust air.

5. Method according to one or more of Patent Claims 1 through 4, **characterized in that** the air admitted through the air intake duct (8) of the chamber (1) is guided through a microfilter in order to improve the sterility.

6. Device for carrying out the method according to one or more of Claims 1 through 5, **characterized in that** a chamber (1) can be acted upon both by cold water or hot water and also by steam from a water/steam unit (16), the cold water/hot water being introduced via spray nozzles (4.1, 4.2, 4.3), the chamber (1) being connected to an outflow (2) via an exhaust air duct (6), and the chamber (1) having an outlet mouth (12) of an openable or closable intake air duct (8) via which the chamber (1) in the closed state can be acted upon by air.

7. Device according to Claim 6, **characterized in that** the outlet mouth of the supply line (14) via which the chamber (1) can be acted upon by steam simultaneously via nozzles (4.1, 4.2, 4.3) is formed either on a roof surface of the chamber (1) or on a back wall or in the lower area or the side walls of the chamber (1).

8. Device according to Claim 6, **characterized in that** automatic shut-off elements which are either spring-controlled or weight-controlled or membrane-controlled or controlled by differential pressure or designed as nonreturn valves (7, 9) are received in the lines (6, 7) for intake air or exhaust air connected to the chamber (1).

9. Device according to Claim 8, **characterized in that** the shut-off elements are designed as forcibly controlled shut-off elements.

## Revendications

1. Procédé de refroidissement d'un produit à nettoyer désinfecté qui est reçu dans une enceinte (1) d'un automate de nettoyage et de désinfection pourvu d'un conduit d'écoulement (2), la désinfection du produit à nettoyer étant effectuée par voie thermique et un programme de lavage ou de nettoyage à séquence variable d'étapes du programme se déroulant à l'intérieur de l'automate de nettoyage et de désinfection, comprenant les étapes de procédé suivantes :
a) le nettoyage final du produit à nettoyer contenu dans l'enceinte (1) est réalisé avec de l'eau à laquelle sont ajoutés des agents auxiliaires,
b) le produit à nettoyer contenu dans l'enceinte (1) est désinfecté thermiquement,
c) après la désinfection thermique du produit à nettoyer, de l'air (29) est introduit de force dans l'enceinte fermée (1) et
**caractérisé en ce que**
d) l'air sortant est évacué de l'enceinte fermée (1) dans le conduit d'écoulement (2), la porte (5) de l'enceinte (1) étant fermée, à travers une conduite d'évacuation (6) pourvue d'une soupape d'échappement (7), le conduit d'écoulement (2) comprenant un coude de siphon (3), l'évacuation de l'air sortant s'effectuant en contournant un volume d'eau faisant fonction de barrage dans le coude de siphon (3).

2. Procédé selon la revendication 1, **caractérisé en ce que**, en cas de durée prolongée d'un processus d'évacuation d'air sortant humide de l'enceinte (1) tandis que la porte (5) est fermée, un séchage supplémentaire du produit à nettoyer contenu dans l'enceinte (1) a lieu.

3. Procédé selon la revendication 1, **caractérisé en ce que** l'air introduit dans l'enceinte (1) après l'étape de désinfection est l'air ambiant.

4. Procédé selon la revendication 1, **caractérisé en ce que**, pour guider l'écoulement d'air d'arrivée respectivement d'air sortant dans la conduite d'évacuation (6) respectivement dans la conduite d'arrivée d'air (8), on prévoit des éléments d'obturation automatiques (7, 9).

5. Procédé selon l'une ou plusieurs des revendications 1 à 4, **caractérisé en ce que** l'air amené à travers la conduite d'arrivée d'air (8) de l'enceinte (1) est guidé à travers un microfiltre pour améliorer l'absence de germe.

6. Dispositif pour l'exécution du procédé selon l'une ou plusieurs des revendications 1 à 5, **caractérisé en ce qu'**une enceinte (1) peut être alimentée aussi bien en eau froide qu'en eau chaude, et également en vapeur d'eau, par une unité eau/vapeur (16), l'entrée d'eau froide/d'eau chaude se faisant par le biais de buses de pulvérisation (4.1, 4.2, 4.3), l'enceinte étant reliée à un conduit d'écoulement (2) par le biais d'une conduite d'évacuation (6) et l'enceinte (1) étant pourvue d'une embouchure (12) d'une conduite d'arrivée d'air (8) pouvant être libérée ou fermée par le biais de laquelle l'enceinte (1) peut, à l'état fermé, être alimentée en air.

7. Dispositif selon la revendication 6, **caractérisé en ce que** l'embouchure de la conduite d'alimentation (14) par le biais de laquelle l'enceinte (1) peut, simultanément, être alimentée en vapeur par le biais de buses (4.1, 4.2, 4.3) est exécutée soit au niveau d'une surface du toit de l'enceinte (1), soit au niveau d'une paroi de limitation postérieure, soit dans la zone inférieure, soit dans les parois de limitation latérales de l'enceinte (1).

8. Dispositif selon la revendication 6, **caractérisé en ce que** les conduites (6, 8) pour l'air d'arrivée ou l'air sortant reliées à l'enceinte (1) contiennent des éléments d'obturation automatiques qui sont exécutés soit avec une commande élastique, soit avec une commande par gravité, soit avec une commande par membrane, soit avec une commande par pression différentielle, soit en tant que clapets antiretour (7, 9).

9. Dispositif selon la revendication 8, **caractérisé en ce que** les éléments d'obturation sont exécutés en tant qu'éléments d'obturation à commande forcée.
